# EUROPEAN PATENT APPLICATION

(11) **EP 2 204 119 A1**
(43) Date of publication of application: **07.07.2010**
(21) Application number: 09180699.2
(22) Date of filing: 23.12.2009
(51) Int. Cl.: A61B 5/0245, A61B 5/16, G08B 21/06

(54) **Drowsiness detection method and apparatus thereof**

(30) Priority: 31.12.2008 US 141861 P
(71) Applicant: Industrial Technology Research Institute, Chutung Hsinchu 31040 (TW)
(72) Inventor: Tao, Teh Ho, Hsinchu City (TW); Su, Yu Jen, 812, Xiaogang District, Kaohsiung City (TW); Huang, Yuan Mei, 330, Taoyuan City, Taoyuan County (TW); Chow, Zu Sho, Jhubei City, Hsinchu County (TW); Chen, Ning Hung, 106, Da-an District Taipei City (TW)
(74) Representative: 2K Patentanwälte Blasberg Kewitz & Reichel

(57) **Abstract**

The apparatus comprises an ultra-wide band module or an electrocardiography module for gathering heartbeat signals of a human being. By sequentially obtaining average heart-rate values of a human being, and according to the features of the heart-rate values over a period of time, the method is utilized to determine whether the human being is going to a state of drowsiness.

## Description

### 1. Technical Field

The disclosure relates to a drowsiness detection method and apparatus thereof.

### 2. Background

While driving long distances, as drivers focus attention for lengthy periods of time on the road and the car, a driver can easily become very tired or even fall asleep. In the early stages of drowsiness, the driver may fall asleep for very brief moments. Attention lapses and reduced alertness occur for short periods (less than 30 seconds) but the driver usually awakens with an awareness of danger. However, the driver subsequently feels weary, and continues to drift repeatedly in and out of consciousness until finally falling completely asleep.

In addition, persons working under highly dangerous conditions, e.g., analysts dealing with dangerous materials analysis, are likely to become lethargic in a very short time in quiet environments requiring highly focused attention. People who become drowsy while working under such conditions cannot pay full attention to dangers in their surroundings.

U.S. patent No. 7,088,250 discloses a fatigue-level estimation apparatus to determine a fatigue level of a subject. U.S. patent No. 6,070,098 utilizes an observation of activities related to fatigue and determines a level of fatigue based on a large amount of processed data. U.S. patent No. 4,967,186 utilizes an IR beam to detect the reflectivity of the eyelid for determining levels of fatigue. However, the detected data of a drowsy subject, such as observations of a driver's behavior or the reflectivity of the eyelid, may exhibit similar behaviors to those of an alert subject. Therefore, there is a need to reduce required data processing amount and to detect drowsiness effectively, so as to meet industrial requirements.

### SUMMARY

A drowsiness detection method and apparatus thereof are disclosed, whereby the drowsiness detection is performed according to heartbeat frequencies during a plurality of time intervals.

One embodiment discloses a drowsiness detection method, comprising the steps of: detecting a plurality of physiological feature values of an object and storing the plurality of physiological feature values in a queue; obtaining a plurality of specific values from the queue, wherein the plurality of specific values comprise a first minimum value, a second minimum value, a first maximum value, and a value of a first position; and obtaining a plurality of difference values between the plurality of specific values and comparing the plurality of difference values with at least one threshold value to generate a drowsiness detection result.

Another embodiment discloses a drowsiness detection method, comprising the steps of: detecting a plurality of physiological feature values of an object and storing the plurality of physiological feature values in a queue; obtaining a maximum value, a first minimum value, and a second minimum value of the queue; obtaining a plurality of difference values between the maximum value, the first minimum value, and the second minimum value; and comparing the plurality of difference values with a plurality of threshold values to generate a drowsiness detection result.

Another embodiment discloses a drowsiness detection method, comprising the steps of: detecting a plurality of physiological feature values of an object and storing them to first and second queues; obtaining a first maximum value, a first minimum value, and a second minimum value of the first queue; obtaining a difference value between the first maximum value and the first minimum value and comparing the difference with a first threshold value and obtaining a difference value between the maximum value and the second minimum value and comparing the difference with a second threshold value, respectively, to generate a first comparison result; obtaining a difference value between the second maximum value and the third minimum value of the second queue and comparing the difference with a third threshold value to generate a second comparison result; and generating a drowsiness detection result according to the first and second comparison results.

Another embodiment discloses a drowsiness detection apparatus comprising a signal detection unit and an operation module. The signal detection unit is configured to obtain a plurality of sequential signals of an object during a plurality of time intervals. The operation module is configured to convert the plurality of sequential signals into a plurality of frequencies and to obtain mutual relationships between the plurality of frequencies, so as to generate a drowsiness detection result.

### BRIEF DESCRIPTION OF THE DRAWINGS

The accompanying drawings, which are incorporated in and constitute a part of this specification, illustrate embodiments of the disclosure and, together with the description, serve to explain the principles of the invention.
FIG. 1 is a flowchart illustrating an exemplary embodiment of the drowsiness detection method;
FIG. 2 shows the detail of step S106 in accordance with an exemplary embodiment;
FIG. 3 illustrates a detection result in accordance with an exemplary embodiment:
FIG. 4 shows the detail of step S106 in accordance with another exemplary embodiment;
FIG. 5 illustrates a detection result in accordance with another exemplary embodiment;
FIG. 6 shows the detail of step S106 in accordance with yet another exemplary embodiment;
FIG. 7 illustrates the first condition determining process in step S601 in accordance with one exemplary embodiment;
FIG. 8 illustrates the second condition determining process in step S602 in accordance with one exemplary embodiment;
FIG. 9 illustrates a detection result in accordance with yet another exemplary embodiment;
FIG. 10 illustrates a system block diagram of a drowsiness detection apparatus in accordance with an exemplary embodiment; and
FIG. 11 illustrates a system block diagram of a drowsiness detection apparatus in accordance with another exemplary embodiment.

### DETAILED DESCRIPTION

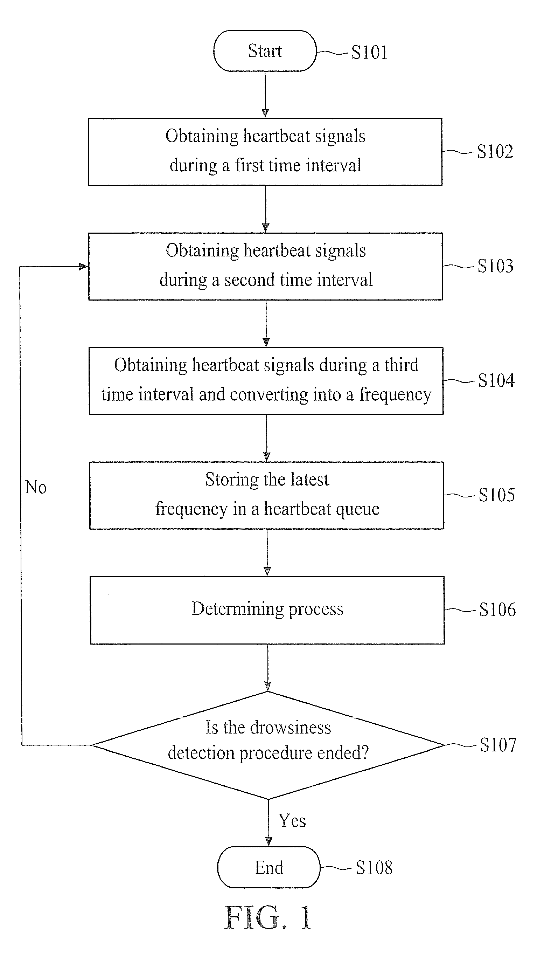

FIG. 1 is a flowchart illustrating an exemplary embodiment of the drowsiness detection method. In step S101, a drowsiness detection procedure is activated. In step S102, heartbeat signals during a first time interval are obtained. The heartbeat signals in the exemplary embodiment are measured with an electrocardiogram machine or an ultra wideband heartbeat detection antenna. In step S103, heartbeat signals during a second time interval are obtained. In step S104, heartbeat signals during a third time interval are obtained, and the peak numbers of the heartbeat signals are converted into a heartbeat frequency. The unit of measure of the heartbeat frequency is "beats per minute." The heartbeat frequency is a physiological feature value in the exemplary embodiment. Also, the physiological feature value can also be a pulse frequency derived from pulse signals. In step S105, the latest heartbeat frequency is stored in a heartbeat queue. In step S106, a determining procedure is performed. Step S107 determines whether the drowsiness detection procedure is ended. If No, the process returns to step S103, while if YES, then the procedure in Step 108 is ended. In the exemplary embodiment, the first time interval is substantially equal to 20 seconds, the second time interval is substantially equal to 10 seconds, and the third time interval is substantially equal to 30 seconds. The latest heartbeat signals gathered during the third time interval (about 30 seconds) comprise heartbeat signals gathered during the second time interval (about 10 seconds) and heartbeat signals gathered within a duration of about 20 seconds prior to the second time interval. In addition, the length of the queue in the exemplary embodiment can be designed according to the gathered frequency for obtaining the heartbeat frequency. The queue in the exemplary embodiment has 20 storage positions, and each of the heartbeat frequencies most recently obtained is stored in the front of the queue, i.e., a first position of the queue. If 20 positions are occupied with the stored heartbeat frequencies, then when a next heartbeat frequency is obtained, the stored value in the first position of the queue is deleted, each of the values stored at other positions in the queue is moved one position before its original position, and the heartbeat frequency most recently obtained is stored in the end of the queue,. The above-mentioned process can be implemented using indexes of the queue.

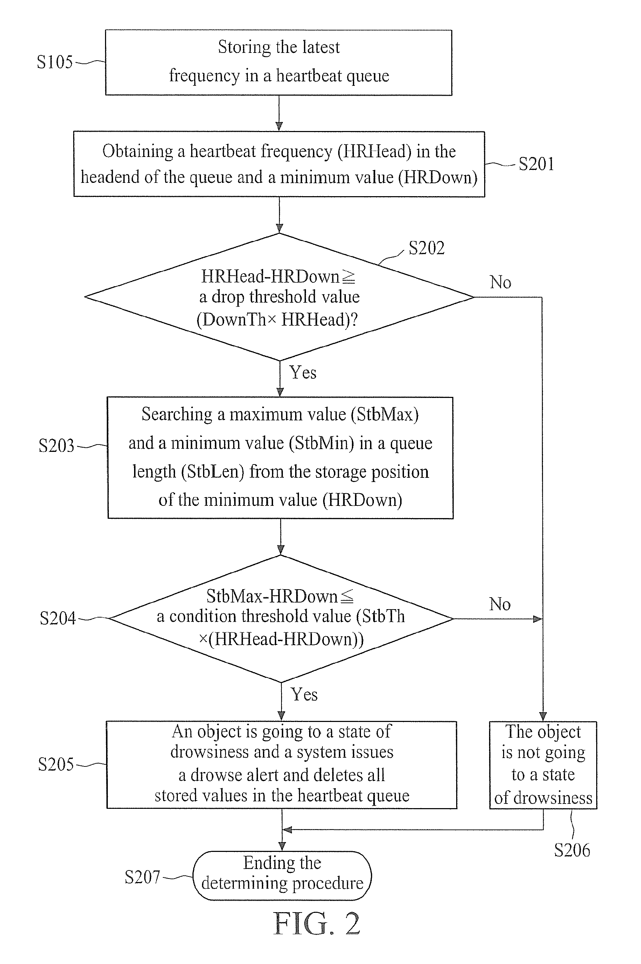

FIG. 2 shows the details of step S106 in accordance with an exemplary embodiment. In step S105, the latest heartbeat frequency is stored in the heartbeat queue. In step S201, a heartbeat frequency HRHead (in the front of the queue), and the value HRDown (the first local smallest value in the queue to appear which is smaller than the previous value and not smaller than the following value in the queue, as the queue is searched from the front to the end) are obtained. A method for searching HRDown is to search from the front of the heartbeat queue to the end of the heartbeat queue and compare every two heartbeat frequencies in the queue until the heartbeat frequency stops decreasing. Step S202 determines whether the difference value between HRHead and HRDown is greater than a drop threshold value. If No, then it is determined that the object is not going to a state of drowsiness (step S206). If YES, then in step S203, a maximum value (StbMax) and a minimum value (StbMin) in a queue length (StbLen) are searched from the storage position of the heartbeat frequency HRDown. Step S204 determines whether the difference value between StbMax and HRDown is less than a condition threshold value. If No, then the object is not going to a state of drowsiness (step S206). If YES, then the object is going to a state of drowsiness and a system issues a drowse alert while deleting all stored values in the heartbeat queue (step S205). In step S207, the determining procedure is ended. In the exemplary embodiment, the drop threshold value = DownTh × HRHead, wherein the value of DownTh is substantially equal to 0.1. In the exemplary embodiment, the length of the StbLen is substantially equal to 6 positions and the condition threshold value = StbTh × (HRHead-HRDown), wherein the value of StbTh is substantially equal to 0.4.

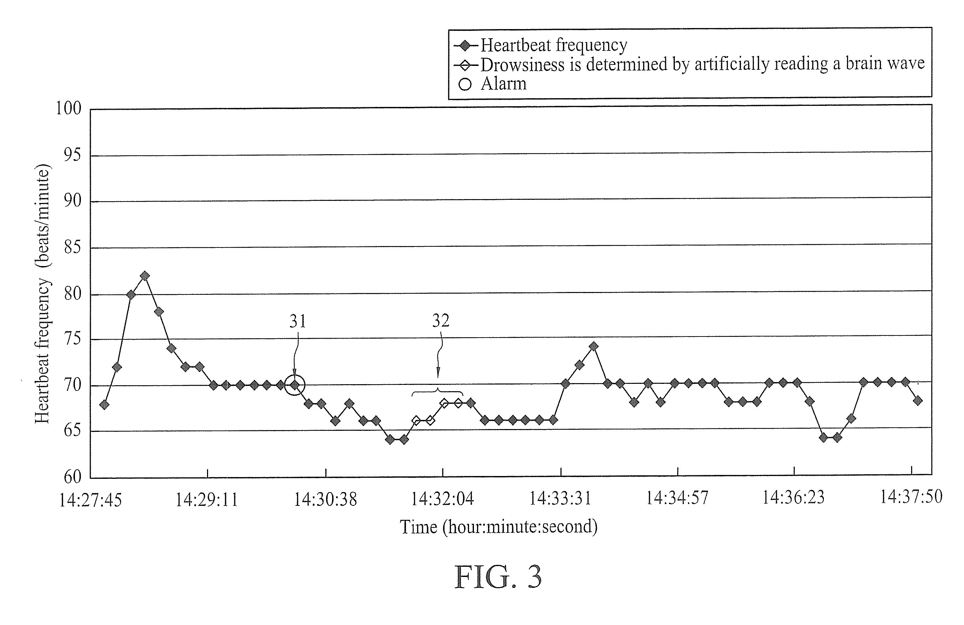

FIG. 3 illustrates a detection result in accordance with an exemplary embodiment. In FIG. 3, the system issues a drowse alert after obtaining a heartbeat frequency 31. A section 32 is a sleeping section obtained by artificially reading a brain wave of the object.

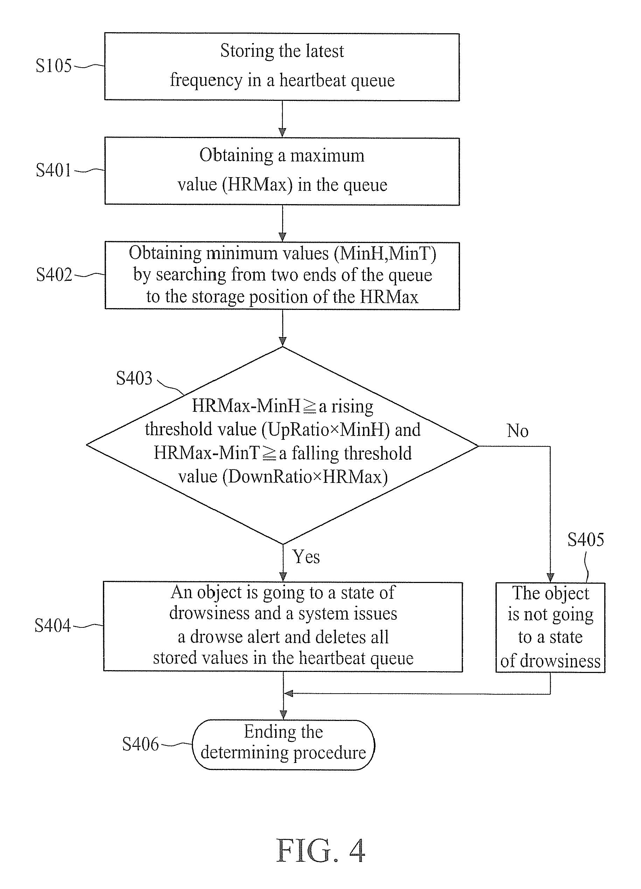

Fig. 4 shows the details of step S106 in accordance with another exemplary embodiment. In step S105, the latest heartbeat frequency is stored in the heartbeat queue. In step S401, a maximum heartbeat frequency (HRMax) in the queue is obtained by searching from the end of the heartbeat queue to the front of the heartbeat queue. In step S402, minimum values MinH and MinT are obtained by searching from the front of the queue to the location of the heartbeat frequency HRMax and from the end of the queue to the location of the heartbeat frequency HRMax, respectively. Step S403 determines whether the difference in value between HRMax and MinH is greater than or equal to a rising threshold value and the difference value between HRMax and MinT is greater than or equal to a falling threshold value. If YES, then the object is going to a state of drowsiness and the system issues a drowse alert and deletes all stored values in the heartbeat queue (step S404). If NO, then the object is not going to a state of drowsiness (step S405). In step S406, the determining procedure is ended. In the exemplary embodiment, the rising threshold value = UpRatio × MinH, wherein the value of the UpRatio is substantially equal to 0.1. In the exemplary embodiment, the falling threshold value = DownRatio × HRMax, wherein the value of the DownRatio is substantially equal to 0.1.

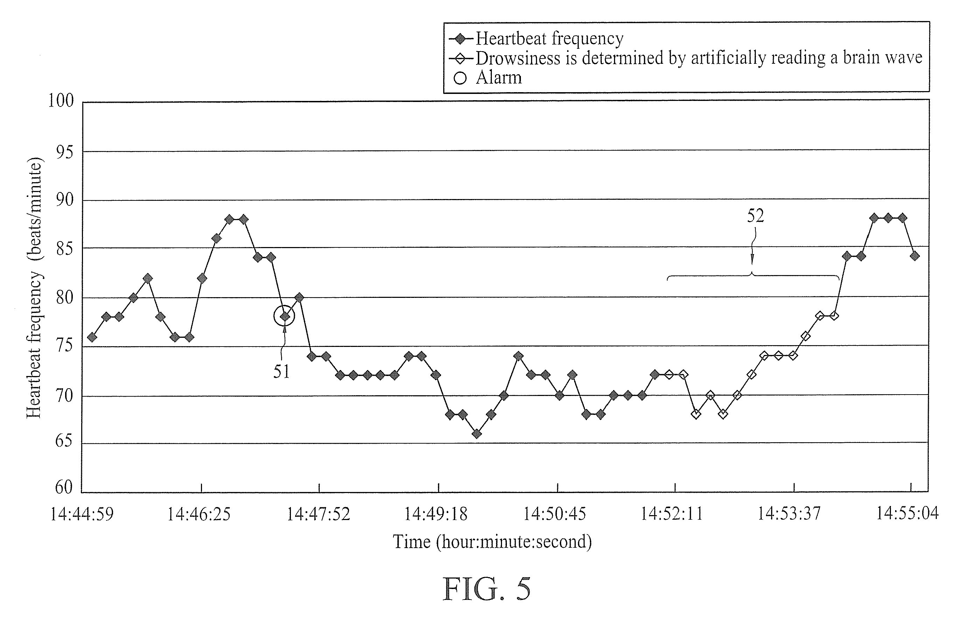

FIG. 5 illustrates a detection result in accordance with another exemplary embodiment. In FIG. 5, the system issues a drowse alert after obtaining a heartbeat frequency 51. A section 52 is a sleeping section obtained by artificially reading a brain wave of the object.

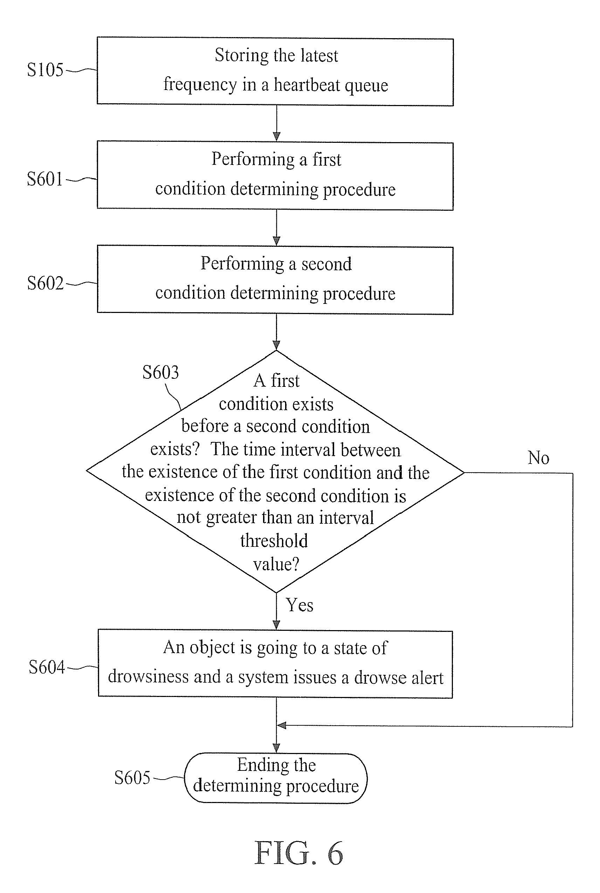

FIG. 6 shows the details of step S106 in accordance with yet another exemplary embodiment. In step S105, the latest heartbeat frequency is stored in the heartbeat queue. In step S601 a first condition determining procedure is performed. In step S601, a second condition determining procedure is performed. Step S603 determines whether a first condition exists before a second condition exists and whether the time interval between the existence of the first condition and the existence of the second condition is not greater than an interval threshold value (the interval threshold value is substantially equal to 3 minutes in the exemplary embodiment). If YES, then it is determined that the object is going to a state of drowsiness and the system issues a drowse alert. In step S605, the determining procedure is ended.

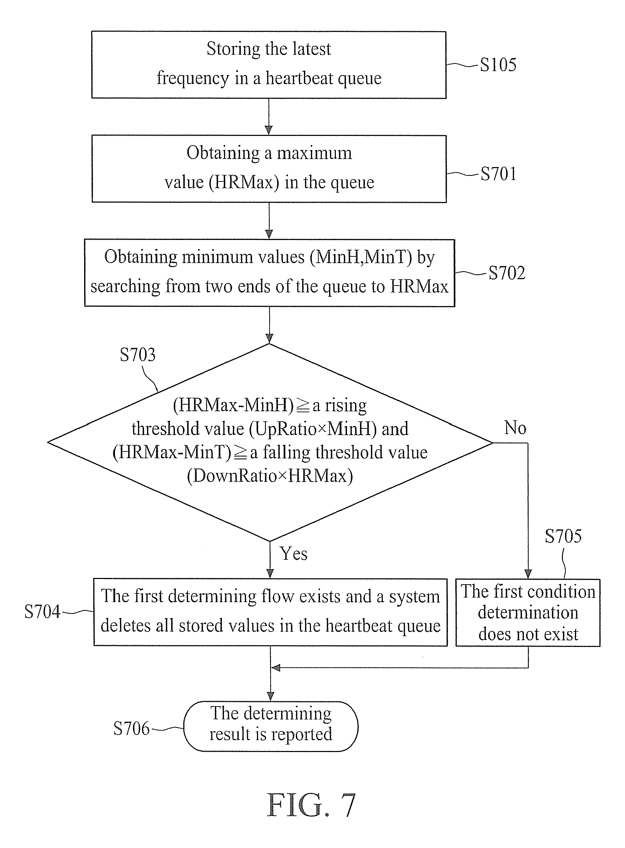

FIG. 7 illustrates the first condition determining procedure in step S601 in accordance with an exemplary embodiment. In step S105, the latest heartbeat frequency is stored in the heartbeat queue. In step S701, a maximum heartbeat frequency (HRMax) in the queue is obtained by searching from the end of the heartbeat queue to the front of the heartbeat queue. In step S702, minimum values MinH and MinT are obtained by searching from the front of the queue to the location of the heartbeat frequency HRMax and from the end of the queue to the location of the heartbeat frequency HRMax, respectively. Step S703 determines whether the difference value between HRMax and MinH is greater than or equal to a rising threshold value and the difference value between HRMax and MinT is greater than or equal to a falling threshold value. If YES, then the first condition determination exists and all stored values in the heartbeat queue are deleted (step S704). If NO, then the first condition determination does not exist (step S705). In step S706, the determining result is reported. In the exemplary embodiment, the rising threshold value = UpRatio × MinH, wherein the value of the UpRatio is substantially equal to 0.1. In the exemplary embodiment, the falling threshold value = DownRatio × HRMax, wherein the value of the DownRatio is substantially equal to 0.1.

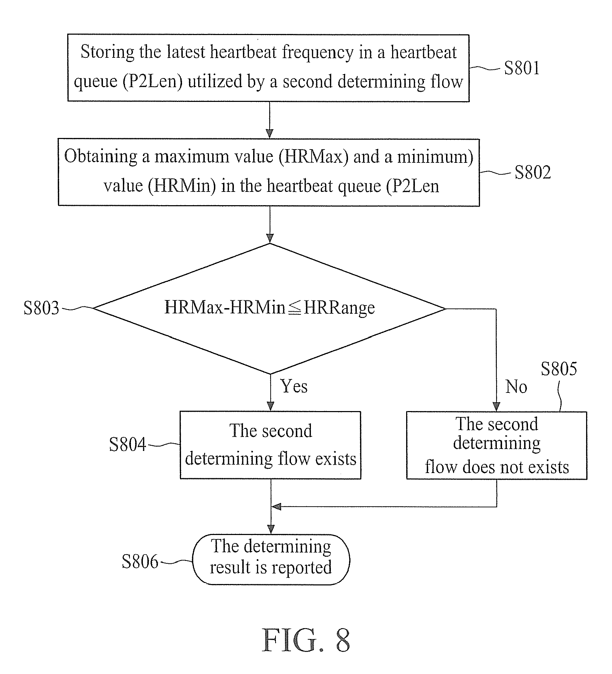

FIG. 8 illustrates the second condition determining procedure in step S602 in accordance with one exemplary embodiment. In step 801, the latest heartbeat frequency is stored in a heartbeat queue (P2Len). In step S802, a maximum value (HRMax) and a minimum value (HRMin) in the queue P2Len are obtained. Step S803 determines whether the difference value between HRMax and HRMin is less than or equal to a range threshold value (HRRange). If YES, then the second condition determination exists (step S804). If NO, then the second condition determination does not exist (step S805). In step S806, the determining result is reported. The queue P2Len is a queue with 6 stored positions in the exemplary embodiment. HRRange is substantially equal to 2 beats per minute.

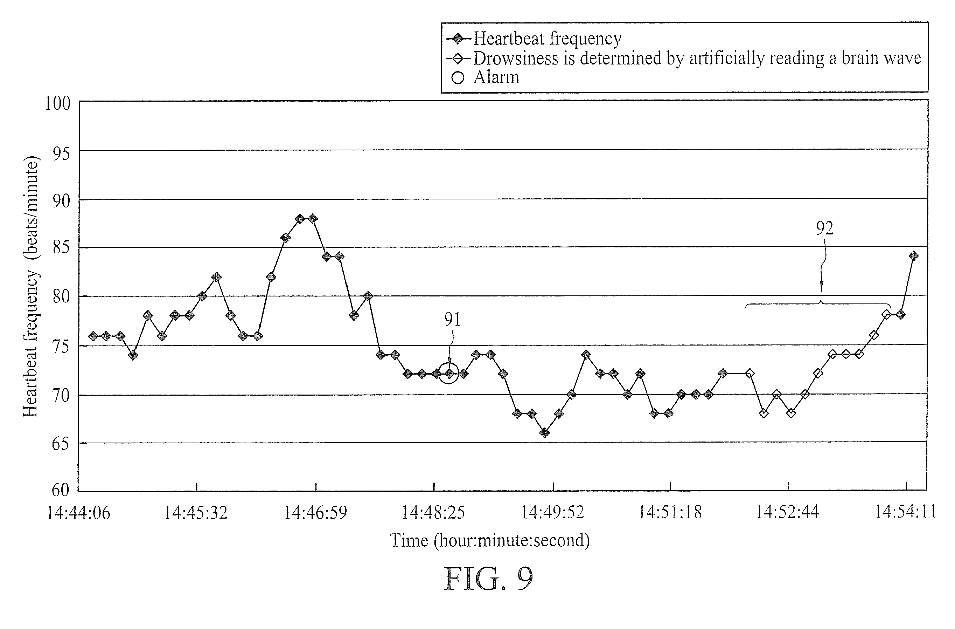

FIG. 9 illustrates a detection result in accordance with yet another exemplary embodiment. In FIG. 9, the system issues a drowse alert after obtaining a heartbeat frequency 91. A section 92 is a sleeping section obtained by artificially reading a brain wave of the object.

In order to enable persons skilled in the art to practice the invention in accordance with an exemplary embodiment, an apparatus of an exemplary embodiment is provided in accordance with the above-mentioned drowsiness detection method.

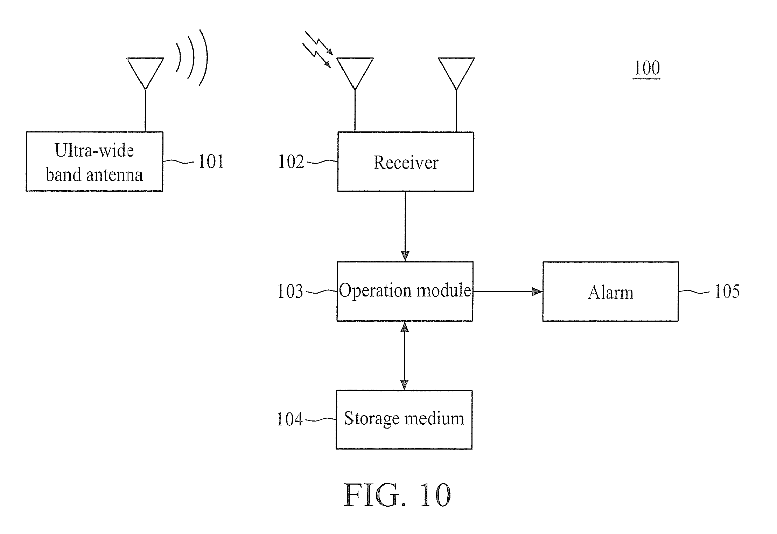

FIG. 10 illustrates a system block diagram of a drowsiness detection apparatus in accordance with an exemplary embodiment. An ultra-wide band signal is emitted to a human body (not shown) by an ultra-wide band antenna 101. A receiver 102 is utilized to receive reflected heartbeat signals after the ultra-wide band signal passes through a human being and is utilized to obtain sequential signals of time intervals during a plurality of time intervals. Periods of the plurality of time intervals can be the same, partially different, or totally different. An operation module 103 is utilized to convert the sequential signals into a heartbeat frequency according to the peak numbers of every set of sequential signals and stores the heartbeat frequency in a storage medium 104. The heartbeat frequency is a physiological feature value in the exemplary embodiment. The physiological feature value can also be a pulse frequency derived from pulse signals. The operation module 103 is utilized to process the relationship of the heartbeat frequency stored in the storage medium 104 and to determine whether an object is going to a state of drowsiness. An alarm 105 is utilized to produce an alarm message according to the output result of the operation module 103.

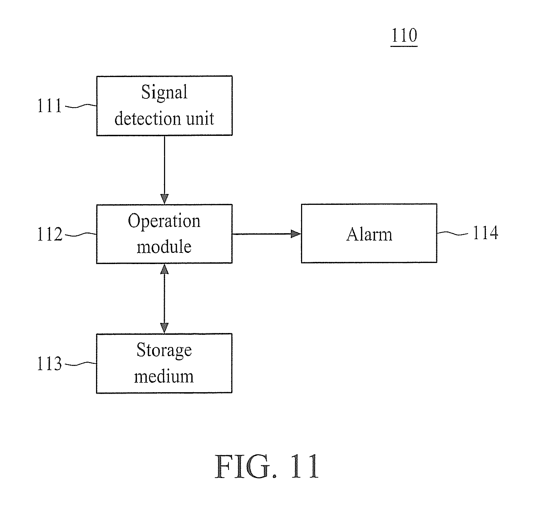

FIG. 11 illustrates a system block diagram of a drowsiness detection apparatus in accordance with another exemplary embodiment. A signal detection unit 111 is utilized to obtain sequential signals of time intervals during a plurality of time intervals. Periods of the plurality of time intervals can be the same, partially different, or totally different. An operation module 112 converts the sequential signals into a heartbeat frequency according to the peak numbers of every set of sequential signals and stores the heartbeat frequency in a storage medium 113. The heartbeat frequency is a physiological feature value in the exemplary embodiment. The physiological feature value can also be a pulse frequency derived from pulse signals. The operation module 112 is utilized to process the relationship of the heartbeat frequency stored in the storage medium 113 and to determine whether an object is going to a state of drowsiness. An alarm 114 is utilized to produce an alarm message according to the output result of the operation module 112.

The above-described exemplary embodiments are intended to be illustrative only. Those skilled in the art may devise numerous alternative embodiments without departing from the scope of the following claims.

## Claims

1. A drowsiness detection method, comprising:
detecting a plurality of physiological feature values of an object and storing the plurality of physiological feature values into at least one queue;
obtaining a plurality of specific values from the queue; and
obtaining a plurality of difference values between the plurality of specific values and comparing the plurality of difference values with at least one threshold value to generate a drowsiness detection result.

2. The method of Claim 1, wherein the plurality of physiological feature values are obtained according to peak numbers of a plurality of sequential signals of the object.

3. The method of Claim 1 or 2, wherein the plurality of specific values comprise a first minimum value, a second minimum value, a first maximum value, and a value of a first position and wherein the first minimum value is a first local minimum value found from the front of the queue and/or
wherein the second minimum value is a minimum value obtained within a queue length by searching from a position of the first minimum value of the queue and/or
where the first maximum value is a maximum value obtained within a queue length by searching from the position of the first minimum value of the queue.

4. The method of any of the preceding claims, wherein the plurality of difference values comprise a first difference value between the value of the first position and the first minimum value, and a second difference value between the first maximum value and the first minimum value, said method
further comprising a step of generating an alarm message and deleting all stored values in the queue if the first difference value is greater than or equal to a first threshold value and the second difference value is less than or equal to a second threshold value.

5. The drowsiness detection method of claim 1 or 2, wherein:
wherein the plurality of specific values comprise a maximum value, a first minimum value, and a second minimum value in the queue; and
said plurality of difference values are obtained between the maximum value, the first minimum value, and the second minimum value, and
the plurality of difference values are compared with a plurality of threshold values to generate a drowsiness detection result.

6. The method of Claim 5, wherein the first minimum value and the second minimum value are minimum values obtained by searching from the front of the queue to a position of the maximum value and from the end of the queue to the position of the maximum value, respectively.

7. The method of Claim 5 or 6, wherein the plurality of difference values comprise a first difference value and a second difference value, wherein the first difference value is a difference value between the maximum value and the first minimum value and a second difference value is a difference value between the maximum value and the second minimum value, said method
further comprising a step of generating an alarm message and deleting all the stored values of the queue if the first difference value is greater than or equal to a first threshold value and the second difference value is smaller than or equal to a second threshold value.

8. The drowsiness detection method of claim 1 or 2, wherein
the plurality of physiological feature values are stored in first and second queues;
said plurality of specific values comprise a first maximum value, a first minimum value, and a second minimum value of the first queue; and
said step of obtaining a plurality of difference values comprises
a) obtaining a difference value between the first maximum value and the first minimum value and comparing the difference with a first threshold value and obtaining a difference value between the maximum value and the second minimum value and comparing the difference with a second threshold value, respectively, to generate a first comparison result; and
b) obtaining a difference value between a second maximum value and a third minimum value of the second queue and comparing the difference with a third threshold value to generate a second comparison result;
in which method a drowsiness detection result is generated according to the first and second comparison results.

9. The method of Claim 8, wherein
the first maximum value is a maximum value of the first queue; and/or
the first minimum value and the second minimum value are minimum values obtained by searching from the front of the first queue to a position of the first maximum value and from the end of the first queue to the position of the first maximum value, respectively; and/or
the second maximum value is a maximum value of the second queue; and/or
the third minimum value is a minimum value of the second queue.

10. The method of Claim 8 or 9, further comprising
a step of generating a first comparison result and deleting all the stored values of the first queue if a difference value between the first maximum value and the first minimum value is greater than or equal to the first threshold value while a difference value between the first maximum value and the second minimum value is greater than or equal to the second threshold value; and
generating a second comparison result if a difference value between the second maximum value and the third minimum value is less than or equal to the third threshold value.

11. The method of Claim 10, further comprising a step of generating an alarm message when the first comparison result is generated before the second comparison result while the period of the first and second comparison results is less than or equal to a time interval.

12. The method of any of the preceding claims, wherein the plurality of physiological feature values are a plurality of heartbeat frequencies or a plurality of pulse frequencies.

13. A drowsiness detection apparatus, comprising:
a signal detection unit configured to obtain a plurality of sequential signals of an object during a plurality of time intervals; and
an operation module configured to convert the plurality of sequential signals into a plurality of frequencies and to obtain mutual relationships of the plurality of frequencies, so as to generate a drowsiness detection result.

14. The apparatus of Claim 13, further comprising a storage medium configured to store the plurality of frequencies and an alarm configured to generate a plurality of messages according to the drowsiness detection result, wherein the operation module is configured to convert peak numbers of the plurality of sequential signals into the plurality of frequencies.

15. The apparatus of Claim 14, wherein periods of the plurality of time intervals can be the same, partially different, or totally different.

16. The apparatus of any of claims 13 to 15, wherein
the plurality of frequencies are a plurality of heartbeat frequencies or a plurality of pulse frequencies, or
wherein the plurality of frequencies are average frequencies during the plurality of time intervals.

17. The apparatus of any of claims 14 to 16, wherein the signal detection unit comprises:
an ultra-wide band antenna configured to emit a plurality of ultra-wide band signals; and
a receiver configured to receive reflected signals after the plurality of ultra-wide band signals pass through the object and to obtain the plurality of sequential signals during the plurality of time intervals; or
wherein the signal detection unit comprises an electrocardiography module.
